# EUROPEAN PATENT SPECIFICATION

(11) **EP 0 668 351 B1**
(45) Date of publication and mention of the grant of the patent: **22.09.1999**
(21) Application number: 95101849.8
(22) Date of filing: 12.10.1990
(51) Int. Cl.: C12N 15/16, C07K 14/505, A61K 38/22

(54) **Erythropoietin analogs**
Erythropoietin- Analoge
Analogues d'érythropoiétine

(30) Priority: 13.10.1989 US 421444; 09.10.1990 WO PCT/US90/05758
(43) Date of publication of application: 23.08.1995
(62) Divisional of application: 90311193.8
(73) Proprietor: Kirin-Amgen, Inc., 6002 Lucerne (CH)
(72) Inventor: Byrne, Thomas Edward, Bethesda, Maryland 20817 (US); Elliott, Steven George, Thousand Oaks, California 91320 (US)
(74) Representative: Brown, John David

(56) References cited:
- EP-A- 0 148 605
- EP-A- 0 370 205
- EP-A- 0 409 113
- JOURNAL OF BIOLOGICAL CHEMISTRY, vol. 263, no. 33, 25 November 1988 MD US, pages 17516-17521, DUBE , S. ET AL. 'Glycosylation at specific sites of erythropoietin is essential for biosynthesis, secretion, and biological function'
- BIOTECHNOLOGY, vol. 6, 1988 NEW YORK US, pages 67-71, GOTO ET AL. 'Production of recombinant human erythropoietin in mammalian cells : host cell dependency of the cloned glycoprotein'

## Description

The present invention relates to erythropoietin analogs, to methods for the preparation of such analogs, and to pharmaceutical compositions comprising such analogs.

### Background of the Invention

Erythropoietin is a glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. Anemia is almost invariably a consequence of renal failure due to decreased production of erythropoietin from the kidney. Recombinant erythropoietin produced by genetic engineering techniques involving the expression of a protein product from a host cell transformed with the gene encoding erythropoietin has been found to be effective when used in the treatment of anemia resulting from chronic renal failure.

Until recently, the availability of erythropoietin has been very limited. Although the protein is present in human urine, excreted levels are too low to make this a practical source of erythropoietin for therapeutic use. Patients suffering from aplastic anemia exhibit elevated levels of urinary erythropoietin relative to healthy individuals, but limited supplies of this urine also make such a source impractical. The purification of human urinary erythropoietin by Miyake et al. in J. Biol. Chem., 252, 5558 (1977), used, as starting material, urine from aplastic anemic individuals.

The identification, cloning, and expression of genes encoding erythropoietin are described in U. S. patent 4,703,008 to Lin. A description of the purification of recombinant erythropoietin from cell medium that supported the growth of mammalian cells containing recombinant erythropoietin plasmids for example, is included in U. S. patent 4,667,016 to Lai et al. The expression and recovery of biologically active recombinant erythropoietin from mammalian cell hosts containing the erythropoietin gene on recombinant plasmids has, for the first time, made available quantities of erythropoietin suitable for therapeutic applications. In addition, knowledge of the gene sequence and the availability of larger quantities of purified protein has led to a better understanding of the mode of action of this protein.

The biological activity of a protein is dependent upon its structure. In particular, the primary structure of a protein (i.e., its amino acid sequence) provides information that allows the formation of secondary (e.g, α helix or β-sheet) and tertiary (overall three-dimensional folding) structures by a polypeptide during and after its synthesis. The disruption of proper secondary and tertiary structures by the introduction of mutations or by chemical or enzymatic treatments can result in a reduction in biological activity.

In procaryotic organisms, the biological activities of proteins are largely governed by the structures described above. Unlike proteins from procaryotic cells, many cell surface and secretory proteins produced by eucaryotic cells are modified with one or more oligosaccharide groups. This modification, referred to as glycosylation, can dramatically affect the physical properties of proteins and can also be important in protein stability, secretion, and subcellular localization. Proper glycosylation can be essential for biological activity. In fact, some genes from eucaryotic organisms, when expressed in bacteria (e.g., E. coli) which lack cellular processes for glycosylating proteins, yield proteins that are recovered with little or no activity by virtue of their lack of glycosylation.

Glycosylation occurs at specific locations along the polypeptide backbone and is usually of two types: O-linked oligosaccharides are attached to serine or threonine residues while N-linked oligosaccharides are attached to asparagine residues when they are part of the sequence Asn-X-Ser/Thr, where X can be any amino acid except proline. The structures of N-linked and O-linked oligosaccharides and the sugar residues found in each type are different. One type of sugar that is commonly found on both is N-acetylneuraminic acid (hereafter referred to as sialic acid). Sialic acid is usually the terminal residue of both N-linked and O-linked oligosaccharides and, by virtue of its negative charge, may confer acidic properties to the glycoprotein.

Both human urinary derived erythropoietin and recombinant erythropoietin (expressed in mammalian cells) having the amino acid sequence 1-165 of human erythropoietin contain three N-linked and one O-linked oligosaccharide chains which together comprise about 40% of the total molecular weight of the glycoprotein. N-linked glycosylation occurs at asparagine residues located at positions 24, 38 and 83 while O-linked glycosylation occurs at a serine residue located at position 126 (Lai et al. J. Biol. Chem. 261, 3116 (1986); Broudy et al. Arch. Biochem. Biophys. 265, 329 (1988)). The oligosaccharide chains have been shown to be modified with terminal sialic acid residues. Enzymatic treatment of glycosylated erythropoietin to remove all sialic acid residues results in a loss of in vivo activity but does not affect in vitro activity (Lowy et al. Nature 185, 102 (1960); Goldwasser et al. J. Biol. Chem. 249, 4202 (1974)). This behavior has been explained by rapid clearance of asialo-erythropoietin from circulation upon interaction with the hepatic asialoglycoprotein binding protein (Morrell et al. J. Biol. Chem. 243, 155 (1968); Briggs, et al. Am. J. Physiol. 227, 1385 (1974); Ashwell et al. Methods Enzymol. 50, 287 (1978)). Thus, erythropoietin possesses in vivo biological activity only when it is sialylated to avoid its binding by the hepatic binding protein.

M. Takeuchi et al, J. Biol. Chem., 263, 1988, 3657-3663 and M. Goto et al, Biotechnology, 6, 1988, 67-71 compare the glycosylation of urinary and recombinant erythropoietin.

The role of the other components in the oligosaccharide chains of erythropoietin is not well defined. It has been shown that non-glycosylated erythropoietin has greatly reduced in vivo activity compared to the glycosylated form but does retain in vitro activity (Dordal et al. Endocrinology 116, 2293 (1985); Lin patent, supra). In another study, however, the removal of N-linked or O-linked oligosaccharide chains singly or together by mutagenesis of asparagine or serine residues that are glycosylation sites sharply reduces in vitro activity of the altered erythropoietin that is produced in mammalian cells (Dube et al. J. Biol. Chem. 263, 17516 (1988)).

Glycoproteins such as erythropoietin can be separated into different charged forms using techniques such as isoelectric focusing (IEF). Several parties have reported IEF studies of crude and partially purified erythropoietin preparations (Lukowsky et al., J. Biochem 50, 909 (1972); Shelton et al. Biochem. Med. 12, 45 (1975); Fuhr et al. Biochem. Biophys. Res. Comm. 98, 930 (1981)). At most, three or four fractions having erythropoietin activity were distinguished by IEF in these studies and none were characterized with respect to carbohydrate content. In addition, no correlation between the isoelectric points of the fractions and their biological activity was made.

During the purification of urinary erythropoietin from human urine discussed in Miyake et. al. supra, two erythropoietin fractions from hydroxylapatite chromatography designated II and IIIA were reported to have the same specific activity. A subsequent carbohydrate analysis of fractions II and IIIA revealed that fraction II had a greater average sialic acid content than fraction IIIA (Dordal et. al. supra).

It is an object of the present invention to provide analogs of human erythropoietin having at least one additional polypeptide site for glycosylation as well as a carbohydrate chain attached to the additional site. Pharmaceutical compositions containing such molecules would have therapeutic benefit.

EP-A-0 370 205 discloses the addition of new carbohydrate chains to hG-CSF and urokinase by introduction of new glycosylation sites.

### Summary of the Invention

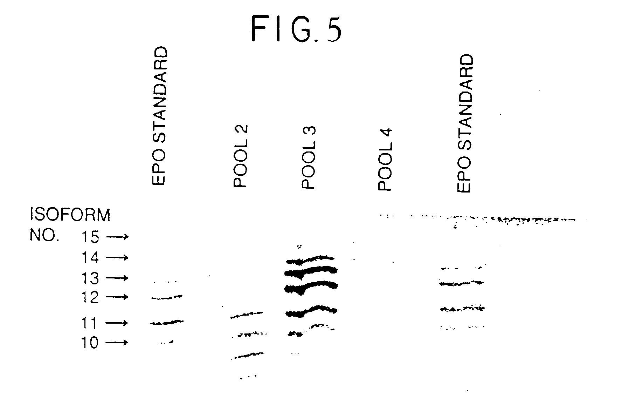

According to the present invention, there is provided an analog of human erythropoietin having one or more changes in the amino acid sequence of human erythropoietin as shown in Figure 5, which change(s) introduce at least one additional polypeptide site for glycosylation, wherein a carbohydrate chain is attached to the additional site, DNA sequences encoding such analogs, a eucaryotic host cell transfected with such DNA sequences, and pharmaceutically acceptable compositions comprising erythropoietin analogs.

The invention also comprises analogs of human erythropoietin having a greater number of sites for carbohydrate chain attachment than human erythropoietin, such as [Asn⁶⁹] EPO; [Thr¹²⁵] EPO; [Pro¹²⁴, Thr¹²⁵] EPO; [Asn⁶⁹, Thr⁷¹] EPO and [Ser⁶⁸, Asn⁶⁹, Thr⁷¹] EPO.

### Brief Description of the Drawings

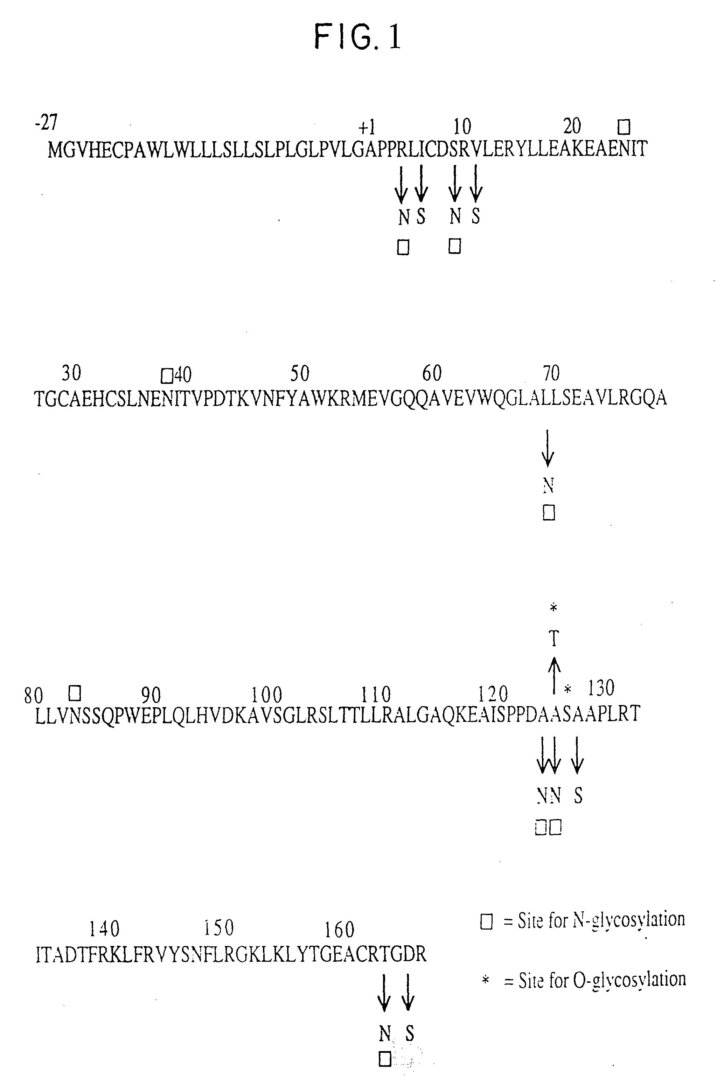

Figure 1 shows the amino acid sequence of human erythropoietin. Squares indicate asparagine residues to which carbohydrate chains are attached and asterisks indicate threonine and serine residues modified with carbohydrate. Additional glycosylation sites provided in the analogs of Example 1 are indicated by mutations to asparagine serine, and threonine.

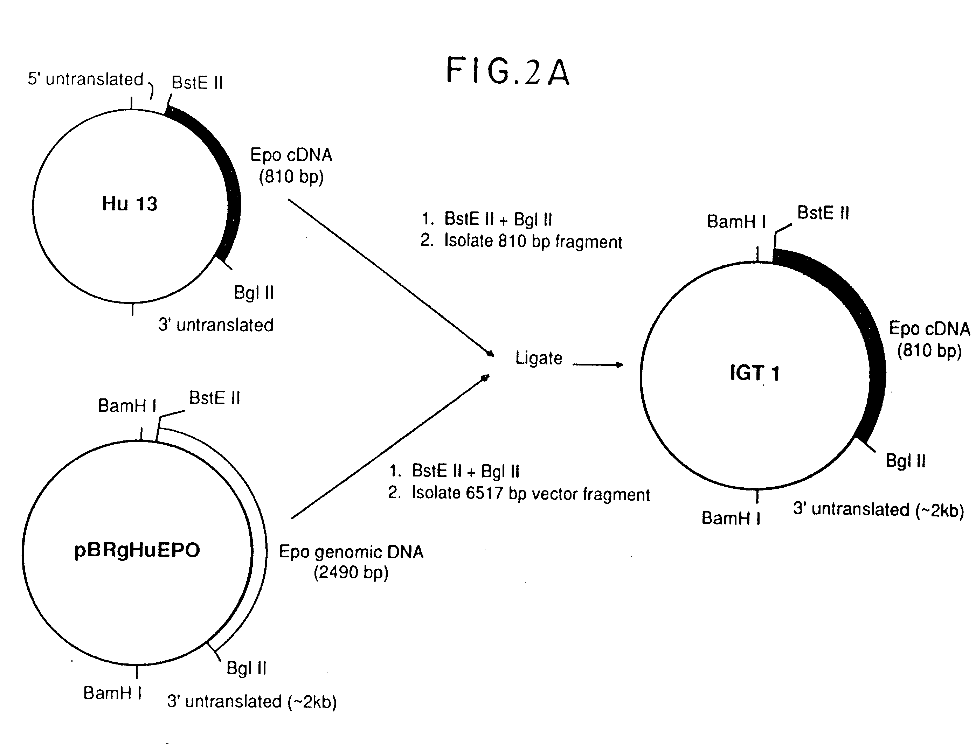

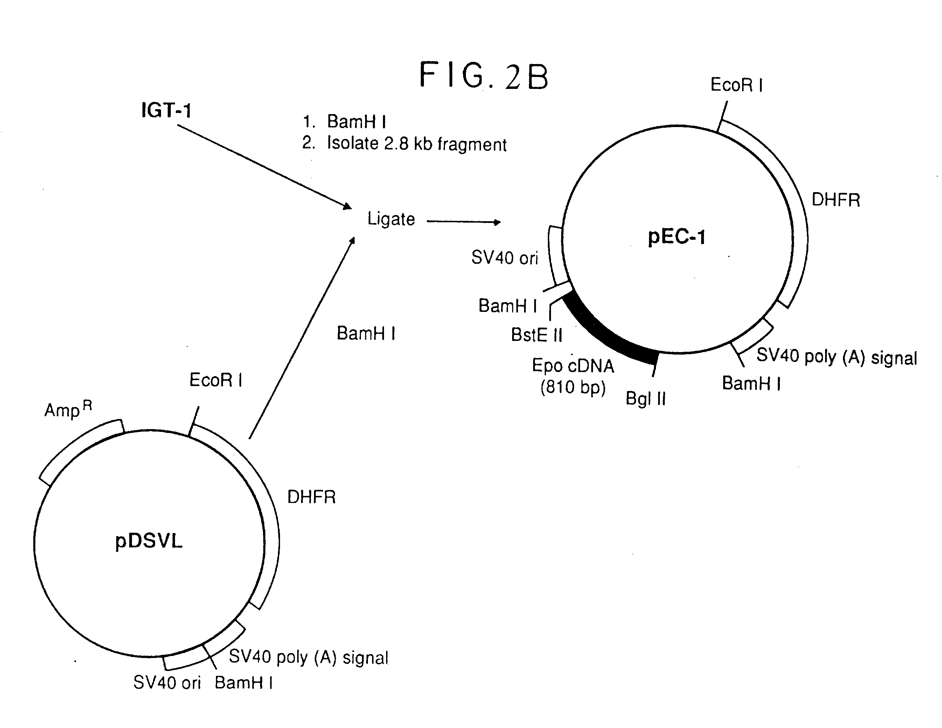

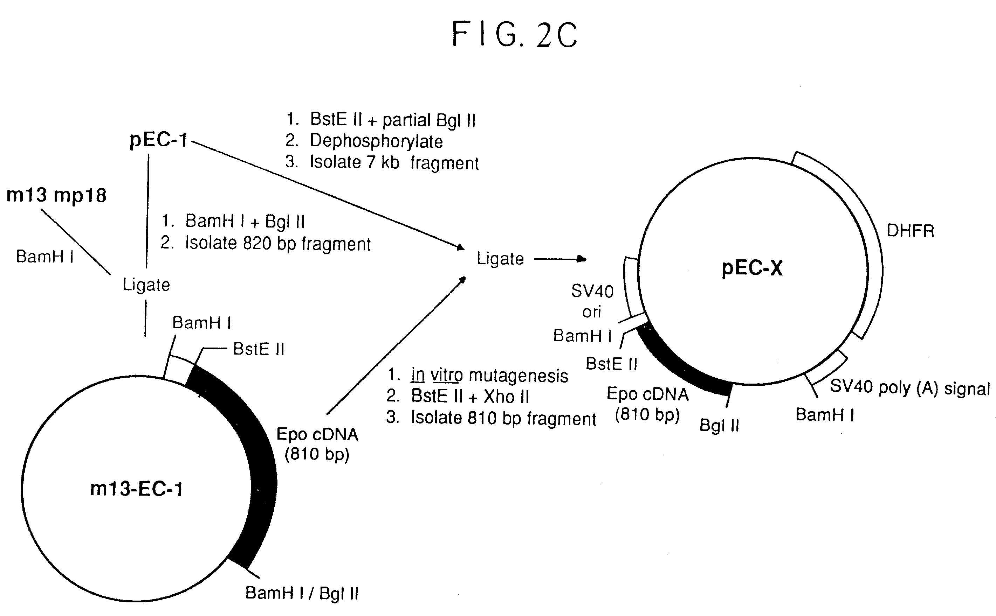

Figures 2A, 2B, and 2C show the series of cloning steps used in generating plasmids for the construction and analysis of analogs of human erythropoietin. These analogs have amino acids altered as shown in Figure 1 which provide additional glycosylation sites.

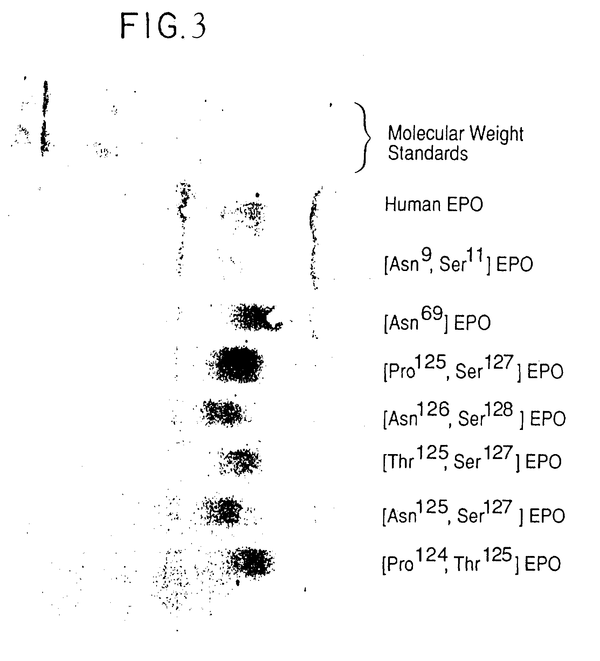

Figure 3 shows a Western blot analysis of COS cell supernatants of human sequence erythropoietin and indicated erythropoietin analogs. The analogs [Asn⁹, Ser¹¹], EPO, [Asn⁶⁹] EPO, [Asn¹²⁵, Ser¹²⁷] EPO, and [Pro¹²⁴, Thr¹²⁵] EPO are constructed as described in Example 6. The analogs [Pro¹²⁵, Thr¹²⁷] EPO, [Asn¹²⁶, Ser¹²⁸] EPO and [Thr¹²⁵, Ser¹²⁷] EPO which do not contain additional carbohydrate chains are shown for comparison.

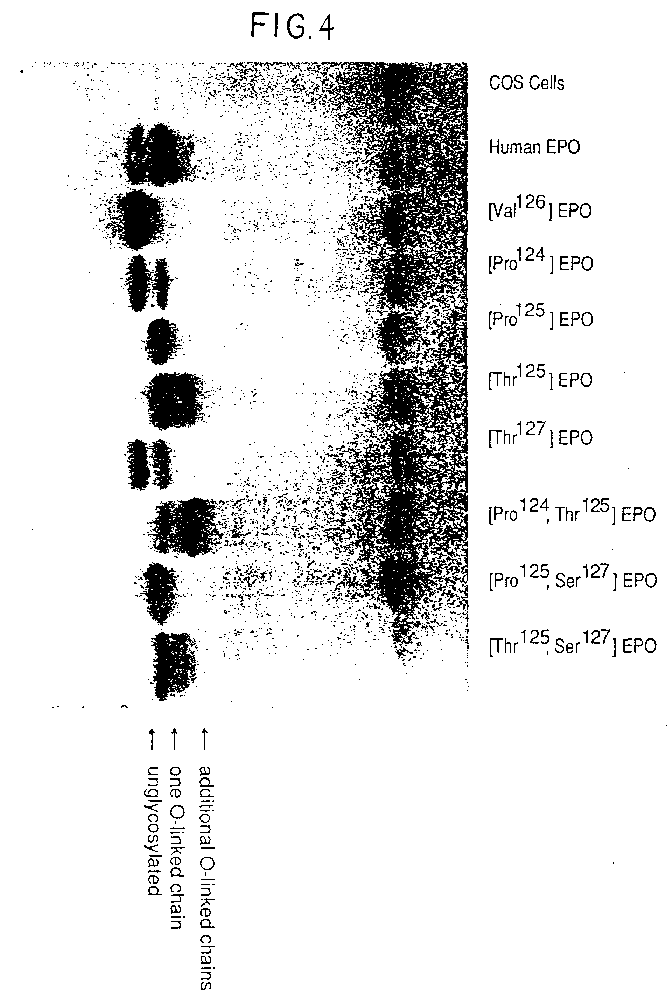

Figure 4 shows a Western blot analysis of COS cell supernatants of human sequences erythropoietin and indicated erythropoietin analogs after treatment with N-glycanase. The analogs [Thr¹²⁵] EPO and [Pro¹²⁴, Thr¹²⁵] EPO are constructed as described in Example 2. The analogs [Val¹²⁶] EPO, [Pro¹²⁴] EPO, [Pro¹²⁵] EPO, Thr¹²⁷] EPO, [Pro¹²⁵, Ser¹²⁷] EPO and [Thr¹²⁵, Ser¹²⁷] EPO as shown for comparison.

Figure 5 shows an isoelectric focusing gel of pools 2, 3 and 4 obtained by Q-Sepharose and C4 reverse phase chromatography of cell medium that supported the growth of CHO cells transfected with erythropoietin cDNA containing the [Thr¹²⁵] mutation. Purified recombinant erythropoietin containing a mixture of isoforms are obtained using procedures described in Example 2 of Lai et al., supra, except that DEAE-Agarose chromatography is replaced by Q-Sepharose chromatography, is also shown in the left and right lanes of the gel.

### Detailed Description of the Invention

Encompassed by the invention are certain analogs of human erythropoietin. As used herein the phrase "analog of human erythropoietin" refers to erythropoietin with one or more changes in the amino acid sequence of human erythropoietin which result in an increase in the number of sites for sialic acid attachment. Analogs are generated by site-directed mutagenesis having additions, deletions, or substitutions of amino acid residues that alter sites that are available for glycosylation. Such analogs have a greater number of carbohydrate chains than human erythropoietin.

Analogs that result in increased biological activity are constructed by increasing the sialic acid content of the erythropoietin molecule. Analogs having levels of sialic acid greater than that found in human erythropoietin are generated by adding glycosylation sites which do not perturb the secondary or tertiary conformation required for biological activity.
Advantageously, the analog of human erythropoietin has 1, 2 or 3 additional sites for N-glycosylation or O-glycosylation. For example, a leucine at position 69 is replaced by an asparagine to give the sequence Asn-Leu-Ser, which serves as a fourth site for N-glycosylation. Such a change can commonly provide up to four additional sialic acids per molecule. Examples of other changes that generate additional N- or O-glycosylation sites are alanines at positions 125 and 127 to asparagine and serine, respectively, alanine at position 125 to threonine and alanines at positions 124 and 125 to proline and threonine, respectively. As will be appreciated by those skilled in the art, the subject invention includes many other analogs of human erythropoietin having additional sites for glycosylation.

Also comprehended by the invention are pharmaceutical compositions comprising a therapeutically effective amount of a specific analog or mixture of analogs together with a suitable diluent, adjuvant and/or carrier useful in erythropoietin therapy. A "therapeutically effective amount" as used herein refers to that amount which provides therapeutic effect for a given condition and administration regimen. The administration of erythropoietin analogs is preferably by parental routes. The specific route chosen will depend upon the condition being treated. The administration of erythropoietin analogs is preferably done as part of a formulation containing a suitable carrier, such as human serum albumin, a suitable diluent, such as a buffered saline solution, and/or a suitable adjuvant. The required dosage will be in amounts sufficient to raise the hematocrit of patients and will vary depending upon the severity of the condition being treated, the method of administration used and the like.

The following examples are offered to more fully illustrate the invention, but are not to be construed as limiting the scope thereof.

### Example 1: Analogs of Human Erythropoietin Having Additional Glycosylation Sites.

### A. Construction of Human Erythropoietin Analogs.

The locations of existing and proposed carbohydrate attachment sites within the erythropoietin amino acid sequence are shown in Figure 1 and the procedure for generating these additional glycosylation sites is summarized in Figures 2A-C and described below.

The following oligonucleotide primers were synthesized for use in in vitro mutagenesis:

[Asn4, Ser6] EPO: 5' CGCCCACCAAACCTCAGCTGTGACAGCCGA 3'
[Asn9, Ser11] EPO: 5' ATCTGTACAACCGAAGCCTGGAGAGGT 3'
[Asn69] EPO: 5' GGGCCTGGCCAACCTGTCGGAAG 3'
[Asn124] EPO: 5' TCCCCTCCAGATAATGCCTCAGCTGC 3'
[Asn125, Ser127] EPO: 5' CAGATGCGAACTCATCTGCTCCAC 3'
[Asn163, Ser165] EPO: 5' AGGCCTGCAGGAATGGGAGCAGATGACCAGGTG 3'
[Thr125] EPO: 5' TCCAGATGCGACCTCAGCTGCTC 3'
[Pro124, Thr125] EPO: 5' CCTCCAGATCCGACCTCAGCTGC 3'

The underlined codons show the mismatched regions where the amino acids indicated in brackets replace the wild-type amino acids.

[Asn⁴, Ser⁶] EPO was constructed to add an N-glycosylation site at Asn 4. [Asn⁹, Ser¹¹] EPO was constructed to add an N-glycosylation site at Asn 9. [Asn⁶⁹] EPO was constructed to add an N-glycosylation site at Asn 69. [Asn¹²⁵, Ser¹²⁷] EPO was constructed to add an N-glycosylation site at Asn 125. [Thr¹²⁵] EPO and [Pro¹²⁴, Thr¹²⁵] EPO were constructed to add an O-glycosylation site at Thr 125.

The following oligonucleotide primers are synthesized for use in in vitro mutagenesis: 
[Asn69, Thr71] EPO:  5' GGGCCTGGCCAACCTGACAGAAGCTGTC 3'

[Ser68, Asn69, Thr71] EPO:
     5' CAGGGCCTGTCCAACCTGACAGAAGCTGTC 3'

[Asn125, Thr127] EPO:  5' CAGATGCGAACTCAACGGCTCCAC 3'

[Asn125, Thr127, Thr131] EPO:
     5'ATGCGAACTCAACGGCTCCACTCACAACAATCACT 3'

[Pro124, Asn125, Ser127] EPO:
     5' CCAGATCCAAATTCATCTGCTCCACTC 3'

[Pro124, Asn125, Thr127] EPO:
     5' CCAGATCCAAATTCAACAGCTCCACTC 3'

[Thr125, Thr126] EPO:  5' CCAGATGCGACAACAGCTGCTCCA 3'
 [Pro¹²⁴, Thr¹²⁵, Thr¹²⁶, Thr¹³¹] EPO:
Starting from [Pro¹²⁴, Thr¹²⁵] EPO cDNA, the oligonucleotide primer 5' AGATCCGACCACCGCTGCTCCAC 3' is used to generate [Pro¹²⁴,Thr¹²⁵, Thr¹²⁶] EPO. The oligonucleotide primer 5'TGCTCCACTCACAACAATCACTG 3' is then used to generate [Pro¹²⁴, Thr¹²⁵, Thr¹²⁶, Thr¹³¹] EPO.

[Asn⁶⁹, Thr⁷¹] EPO and [Ser⁶⁸, Asn⁶⁹, Thr⁷¹] EPO are constructed to add an N-glycosylation site at Asn 69 and to enhance N-glycosylation at that site. [Asn¹²⁵, Thr¹²⁷] EPO, [Asn¹²⁵, Thr¹²⁷, Thr¹³¹] EPO, [Pro¹²⁴, Asn¹²⁵, Ser¹²⁷] EPO and [Pro¹²⁴, Asn¹²⁵, Thr¹²⁷] EPO are constructed to add an N-glycosylation site at Asn 125 and to increase glycosylation at that site. [Thr¹²⁵, Thr¹²⁶] EPO and [Pro¹²⁴, Thr¹²⁵, Thr¹²⁶, Ser¹³¹] EPO are constructed to add an O-glycosylation site at Thr 125 and to increase glycosylation at that site.

The source of erythropoietin DNA for in vitro mutagenesis was plasmid Hu13, a human erythropoietin cDNA clone in pUC 8 (Law et al. Proc Natl. Acad. Sci. 83, 6920 (1986)). Plasmid DNA derived from Hu13 was digested with BstEII and BglII restriction enzymes, the resulting DNA fragments were subjected to agarose gel electrophoresis, and the 810 base pair (bp) erythropoietin DNA fragment was isolated from the gel using a GeneClean™ kit and procedures supplied by the manufacturer (BIO 101, Inc.). Plasmid pBRgHuEPO contains the erythropoietin genomic gene as a BamHI fragment inserted into a derivative of pBR322, as described in commonly owned Lin patent, supra. pBRgHuEPO was also digested with BstEII and BglII and the 6517 bp vector fragment was recovered. Ligation of the two fragments results in IGT1. To construct pEC-1, pDSVL (described in commonly owned Lin patent, supra, and shown in Figure 1B) was digested with BamHI and the isolated 2.8 kilobase (kb) BamHI fragment from IGT1 containing erythropoietin cDNA was ligated into it.

In order to generate single-stranded DNA for in vitro mutagenesis, pEC-1 was digested with BamHI and BglII and the 820 bp erythropoietin cDNA fragment was isolated. It was ligated into the BamHI site of m13mp18 to give m13-EC-1. Single stranded DNA was recovered from supernatants of E. coli strain RZ1032 infected by m13-EC-1 as described by Kunkel et al. Methods in Enzymol. 154, 367 (1987) and Messing, Methods in Enzymol. 101, 20 (1983). For in vitro mutagenesis approximately 1 µg of single-stranded DNA and 0.2 pmole of one of the synthetic primers described above were mixed with 6 ml of buffer (250 mM Tris pH 7.8, 50 mM MgCl₂, and 50 mM dithiothreitol). For annealing of the primer to the template, the reaction volume was adjusted to 10 µl with water, the mixture was heated to 65°C for 5 minutes and then allowed to cool to room temperature. For the elongation reaction 2.5 ml of each of dTTP, dATP, dGTP, dCTP and ATP (all at 10 µM) were added, followed by 1 µl (1 unit) of E. coli DNA polymerase (Klenow fragment) and 1 µl (1 unit) of T4 DNA ligase. The mixture was then incubated overnight at 14°C and used to transform E. coli JM 109 (Yanisch-Perron et al. Gene 33, 103 (1985)) as described (Messing, supra).

To identify mutant clones by differential hybridization, plaques on nutrient agar were transferred to Gene Screen filters (New England Nuclear). The filters were dried under a heat lamp and then incubated for one hour in 6x SSC containing 1% SDS at 60°C . For the hybridization, the oligonucleotide primer above (8 pmoles) was end-labeled with T4 polynucleotide kinase and γ ³²P-labeled ATP and incubated with the filters overnight in 6x SSC, 0.5% SDS and 100 mg/ml salmon sperm DNA at 37°C for the [Asn¹²⁴] mutation, 55°C for the [Asn⁴, Ser⁶] mutation, 65°C for the [Thr¹²⁵] and the [Pro¹²⁴, Thr¹²⁵] mutations, and 70°C for the [Asn⁹, Ser¹¹] and [Asn¹⁶³, Ser¹⁶⁵] mutations. The next day, the filters were washed three times with 6x SSC at room temperature and subjected to autoradiography. If necessary, the filters were then washed with 6x SSC at increasing temperatures until little or no hybridization was detected to plaques having the wild-type erythropoietin cDNA sequence. Clones that gave positive hybridization signals under these conditions were identified and retransfected into JM109 to isolate a pure clone. Dideoxy chain termination sequence analysis indicated that the mutations to asparagine, serine threonine and proline residues were present.

Double stranded m13 EC-1 DNAs carrying the [Asn⁴, Ser⁶], [Asn⁹, Ser¹¹], [Asn⁶⁹], [Asn¹²⁴], [Asn¹²⁵], [Ser¹²⁷], [Asn¹⁶³, Ser¹⁶⁵] [Thr¹²⁵], and [Pro¹²⁴, Thr¹²⁵] changes were recovered from JM109 transfected cells by the boiling method (Holmes et al. Anal. Biochem 117, 193 (1981)). The DNAs were digested with BstEII and XhoII and the 810 bp erythropoietin DNA fragments were isolated. pEC-1 were digested with BstEII followed by a partial digestion with BglII and the 5' termini of the resulting fragments are dephosphorylated with bacterial alkaline phosphatase in 10 mM Tris, pH 8 at 60°C for 60 minutes. The 7 kb vector fragment lacking the 810 bp BstEII-BglII fragment was isolated and ligated to the erythropoietin fragments above. The resulting plasmids (designated pEC-X where X identifies the particular mutation) contain human erythropoietin with altered amino acid residues at the indicated positions.

cDNA clones of the human erythropoietin sequence and analogs corresponding to [Asn4, Ser6], [Asn9, Ser11], [Asn⁶⁹], [Asn¹²⁴], [Asn¹²⁵, Ser¹²⁷], [Asn¹⁶³, Ser¹⁶⁵], [Thr¹²⁵] and [Pro¹²⁴, Thr¹²⁵] erythropoietin cDNA clones were transferred into COS-1 cells (ATCC No. CRL-1650) by electroporation. COS-1 cells were harvested from semi-confluent dishes, washed with medium (Dulbecco's modified essential medium containing 5% fetal calf serum and 1% L-glutamine/penicillin/ streptomycin (Irvine Scientific)) and resuspended at 4 x 10⁶ cells/ml. One ml of cells was transferred to an electroporation cuvette (Bio-Rad) and electroporated with a Bio-Rad Gene Pulser™ at 25 µFarads and 1600 volts in the presence of 100 to 200 µg of carrier DNA and 2 to 20 µg of plasmid DNA encoding the erythropoietin analog. The electroporated cells were plated at 2 x 106 cells per 60 mm tissue culture dish in 5 ml of medium. Two to four hours after plating the medium was replaced with 5 ml of fresh medium. The conditioned medium was collected 3 to 5 days after electroporation.

### B. Assays for erythropoietin analog activity

RIAs were performed according to Egrie et al., (Immunobiology 172, 213, (1986). The in vivo biological activity of erythropoietin analogs was determined by the exhypoxic polycythemic mouse bioassay (Cotes et al., Nature 191, 1065 (1961)).

In vitro erythropoietin activity was determined by the erythroid colony forming assay as described by Iscove et al. J. Cell Physiol. 83, 309-320 (1974) with modifications. The mononucleated cells from human bone marrow cells were partially purified on a ficoll-paque cushion and washed in Iscove medium before plating to remove the adherent cells. The culture medium contained 0.9% methyl cellulose and did not include any bovine serum albumin. The erythroid colonies are scored after 8 to 10 days of culture.

The erythropoietin analogs transfected and expressed in COS cells as described in Section A were analyzed in crude COS cell supernatants by RIA and by the erythroid colony forming assay. Human sequence erythropoietin has an in vitro activity that is comparable to the RIA activity as determined by the above-mentioned assays. The analogs [Asn⁶⁹] EPO, [Asn¹²⁵, Ser¹²⁷] EPO, [Thr¹²⁵] EPO and [Pro¹²⁴, Thr¹²⁵] EPO exhibited an in vitro activity that is comparable to the RIA activity and gave evidence of having additional carbohydrate chains (as determined in Section C). These analogs are analyzed further by transfecting a cDNA clone encoding the erythropoietin analog into CHO cells, purifying the erythropoietin analog and measuring the in vivo biological activity of the purified analog.

### C. Western Blot Analysis

A volume of supernatant containing 5-20 units from COS cells transfected with erythropoietin analog cDNAs as described in Section A was immunoprecipitated overnight at room temperature with a rabbit anti-erythropoietin polyclonal antibody. 20-80 µl of 1:1 Protein A-Sepharose in phosphate buffered saline (PBS) was added to the immunoprecipitate and allowed to incubate for one hour at room temperature. The samples were centrifuged, washed with PBS and, where indicated, the pellet was treated with N-glycanase to remove N-linked carbohydrate chains. The samples were analyzed by 15% SDS-polyacrylamide gel electrophoresis, transferred to nitrocellulose and subjected to Western analysis as described (Burnette et al. Anal. Biochem. 112, 195-203 (1981); Elliot et al. Gene 79, 167-180 (1989)) using a mixture of mouse anti-erythropoietin monoclonal antibodies. One such antibody, 9G8A, is described in Elliot et al. (1989) Blood 74, Supp. 1, A. 1228.

Analysis of COS cell supernatants transfected with [Asn⁶⁹] EPO and [Asn¹²⁵, Ser¹²⁷] EPO cDNA revealed increased protein size compared to human sequence erythropoietin. This increased size is indicative of an additional N-linked carbohydrate chain (Figure 7). Treatment of supernatants from COS cells transfected with [Thr¹²⁵] EPO and [Pro¹²⁴, Thr¹²⁵] EPO cDNA with N-glycanase revealed an increased protein size compared to human sequence erythropoietin. This increased size is indicative of an additional O-linked carbohydrate chain (Figure 8).

### D. Isolation of Erythropoietin Analog Isoforms

The erythropoietin analog [Thr¹²⁵] EPO was constructed as described in Section A. An 810 bp. erythropoietin cDNA fragment carrying the [Thr¹²⁵] mutation was isolated by cleaving the plasmid pEC containing the [Thr¹²⁵] mutation with BstEII and BglII and ligating the fragment to pDECΔ, a derivative of pDSα2. pDSα2 is generally described in commonly owned U.S. Patent Application No. 501,904, hereby incorporated by reference. pDECΔ was derived from pDSα2 by the following steps:
(1) The HindIII site of pDSα2 was deleted by digesting pDSα2 DNA with HindIII, treating the HindIII cohesive ends with E. coli DNA Polymerase (Klenow fragment) and dNTPs, and religating the blunt-ended vector. The resulting plasmid was pDSα2ΔH.
(2) pDSα2ΔH was digested with SalI and a synthetic oligonucleotide having an SV40 splice signal with a SalI linker attached to the 3' end of the splice signal was ligated to it. The synthetic oligonucleotide had the following sequence: 
5' TCGAGGAACTGAAAAACCAGAAAGTTAACTGGTAAGTTTAGT

CTTTTTGTCTTTTATTTCAGGTCCCGGATCCGGTGGTGCAAATCA

AAGAACTGCTCCTCAGTGGATGTTGCCTTTACTTCTAGGCCTGTACGG

AAGTGTTACTTCTGCTCTAAAAGCTGCTGCAACAAGCTGGTCGACC 3'
 The resulting plasmid was pDSα2ΔH splice.
3) pDSα2ΔH splice was digested with SalI and blunt-ended by treating the cohesive ends with T4 DNA polymerase and dNTPs. An 820 bp. BamHI-BglII human erythropoietin cDNA fragment was blunt-ended by the same method and ligated to the plasmid. The resulting plasmid was pDEC.
4) pDEC was digested with KpnI and PvuII and blunt-ended by treating the cohesive ends with mung bean nuclease. The plasmid was religated to delete the excised KpnI-PvuII fragment resulting in the plasmid pDECΔ.

Plasmid pDECΔ containing [Thr¹²⁵] erythropoietin cDNA was transfected into DHFR-deficient CHO cells. 770 ml of CHO cell conditioned medium was concentrated using a 10,000 dalton molecular weight cutoff membrane and diafiltered against 10 mM Tris-HCl, pH 8.6 to a final volume of 34 ml. A 17 ml. aliquot of the concentrate was loaded onto a Q-Sepharose fast flow column (5 ml bed volume) equilibrated in the same buffer and eluted in a linear gradient of 0-250 mM NaCl in 10 mM Tris-HCl, pH 8.6. Aliquots of column fractions, either untreated or digested with N-glycanase, were analyzed by SDS-PAGE or IEF and pools (designated 2, 3 and 4) were made based upon the isoform and/or carbohydrate composition of the fractions. Each pool was loaded onto a Vydac C4 column (214TPB 2030; 1 cm diameter; 1.8-2.5 ml bed volume; 0.34 ml/min) and washed with two column volumes of 20% ethanol in 10 mM Tris-HCl, pH 7.0. The columns were eluted with linear gradients of 20-94% ethanol, 10 mM Tris, pH 7.0. Pools were made, diluted into 10 mM Tris-HCl, pH 7.0, and loaded onto Q-Sepharose fast flow columns. Following a wash in 10 mM Tris-HCl, pH 7.0, the samples were eluted with 20 mM sodium citrate, 250 mM NaCl, pH 7.0. The purified [Thr¹²⁵] pools were analyzed by IEF and are shown in Fig. 5. EPO analog is analyzed for in vivo biological activity as described above (Cotes et al., supra).

The present Application (90311193.8) generally describes methods relating to the isolation and assay of erythropoietin having specific numbers of sialic acids per molecule.

## Claims

1. An analog of human erythropoietin having one or more changes in the amino acid sequence of human erythropoietin, as shown in Figure 5, which change(s) introduce at least one additional polypeptide site for glycosylation, wherein a carbohydrate chain is attached to the additional site.

2. The analog of Claim 1 wherein an/the additional site is for an N-linked carbohydrate chain.

3. The analog of Claim 1 wherein an/the additional site is for an O-linked carbohydrate chain.

4. The analog of Claim 1 wherein the changes in the amino acid sequence are additions, deletions or substitutions of amino acid residues.

5. The analog of Claim 2 wherein an/the additional site is substituted at position 69 of the amino acid sequence of human erythropoietin as shown in Figure 5.

6. The analog of Claim 3 wherein the site is substituted at position 125 of the amino acid sequence of human erythropoietin as shown in Figure 5.

7. The analog of Claim 1 wherein an additional carbohydrate chain provides sites for sialic acid attachment.

8. The analog of any one of the preceding claims, which is the product of expression of an exogenous DNA sequence.

9. An analog according to Claim 1 selected from the group consisting of:
Asn⁶⁹ EPO;
Asn⁶⁹, Thr⁷¹ EPO;
Ser⁶⁸, Asn⁶⁹, Thr⁷¹ EPO;
Thr¹²⁵ EPO; and
Pro¹²⁴, Thr¹²⁵ EPO.

10. A DNA sequence encoding an analog of human erythropoietin of any one of the preceding claims.

11. A eucaryotic host cell transfected with a DNA sequence of claim 10 in a manner allowing the host cell to express an analog of human erythropoietin.

12. A composition comprising a therapeutically effective amount of an erythropoietin analog of any one of Claims 1 to 9, together with a pharmaceutically acceptable diluent, adjuvant or carrier.

## Patentansprüche

1. Ein Analog von menschlichem Erythropoietin mit einer oder mehreren Änderungen in der Aminosäuresequenz von menschlichem Erythropoietin, wie in Figur 5 gezeigt, wobei die Änderung(en) wenigstens eine zusätzliche Polypeptidstelle für Glycosylierung einführt/einführen und wobei eine Kohlenhydratkette an die zusätzliche Stelle gebunden ist.

2. Das Analog von Anspruch 1, wobei eine/die zusätzliche Stelle für eine N-gebundene Kohlenhydratkette ist.

3. Das Analog von Anspruch 1, wobei eine/die zusätzliche Stelle für eine O-gebundene Kohlenhydratkette ist.

4. Das Analog von Anspruch 1, wobei die Änderungen in der Aminosäuresequenz Additionen, Deletionen oder Substitutionen von Aminosäureresten sind.

5. Das Analog von Anspruch 2, wobei eine/die zusätzliche Stelle substituiert ist an Position 69 der Aminosäuresequenz von menschlichem Erythropoietin, wie in Figur 5 gezeigt.

6. Das Analog von Anspruch 3, wobei die Stelle substituiert ist an Position 125 der Aminosäuresequenz von menschlichem Erythropoietin, wie gezeigt in Figur 5.

7. Das Analog von Anspruch 1, wobei eine zusätzliche Kohlenhydratkette Stellen für die Bindung von Sialinsäure vorsieht.

8. Das Analog nach einem der vorangehenden Ansprüche, das das Expressionsprodukt einer exogenen DNA-Sequenz ist.

9. Ein Analog entsprechend Anspruch 1, das ausgewählt ist aus der Gruppe, die umfaßt:
Asn⁶⁹ EPO;
Asn⁶⁹ Thr⁷¹ EPO;
Ser⁶⁸, Asn⁶⁹, Thr⁷¹ EPO;
Thr¹²⁵ EPO; und
Pro¹²⁴, Thr¹²⁵ EPO.

10. Eine DNA-Sequenz, die für ein Analog von menschlichem Erythropoietin nach einem der vorangehenden Ansprüche kodiert.

11. Eine eukaryontische Wirtszelle, die mit einer DNA-Sequenz nach Anspruch 10 in einer Art und Weise transfiziert ist, die erlaubt, daß die Wirtszelle ein Analog von menschlichem Erythropoietin exprimiert.

12. Eine Zusammensetzung, die eine therapeutisch wirksame Menge eines Erythropoietin-Analogs nach einem der Ansprüche 1 bis 9 zusammen mit einem pharmazeutisch akzeptablen Verdünnungsmittel, Adjuvans oder Träger umfaßt.

## Revendications

1. Analogue de l'érythropoïétine humaine ayant un ou plusieurs changements dans la séquence d'acides aminés de l'érythropoïétine humaine, comme montré sur la figure 5, changement(s) introduisant au moins un site polypeptidique supplémentaire pour la glycosylation, dans lequel une chaîne carbohydratée est fixée au site supplémentaire.

2. Analogue de la revendication 1, dans lequel un/le site supplémentaire est pour une chaîne carbohydratée N-liée.

3. Analogue de la revendication 1, dans lequel un/le site supplémentaire est pour une chaîne carbohydratée O-liée.

4. Analogue de la revendication 1, dans lequel les changements dans la séquence d'acides aminés sont des additions, des délétions ou des substitutions de résidus d'acides aminés.

5. Analogue de la revendication 2, dans lequel un/le site supplémentaire est substitué en position 69 de la séquence d'acides aminés de l'érythropoïétine humaine comme montré sur la figure 5.

6. Analogue de la revendication 3, dans lequel le site est substitué en position 125 de la séquence d'acides aminés de l'érythropoïétine humaine comme montré sur la figure 5.

7. Analogue de la revendication 1, dans lequel une chaîne carbohydratée supplémentaire fournit des sites pour la fixation d'acide sialique.

8. Analogue selon l'une quelconque des revendications précédentes, qui est le produit de l'expression d'une séquence d'ADN exogène.

9. Analogue selon la revendication 1 choisi à partir du groupe constitué de :
Asn⁶⁹ EPO ;
Asn⁶⁹, Thr⁷¹ EPO ;
Ser⁶⁸, Asn⁶⁹, Thr⁷¹ EPO ;
Thr¹²⁵ EPO ; et
Pro¹²⁴, Thr¹²⁵ EPO.

10. Séquence d'ADN codant un analogue de l'érythropoïétine humaine selon l'une quelconque des revendications précédentes.

11. Cellule hôte eucaryote transfectée par une séquence d'ADN de la revendication 10 d'une manière permettant à la cellule hôte d'exprimer un analogue de l'érythropoïétine humaine.

12. Composition comprenant une quantité thérapeutiquement efficace d'un analogue d'érythropoïétine selon l'une quelconque des revendications 1 à 9, en même temps qu'un support, adjuvant ou diluant pharmaceutiquement acceptable.
